# EUROPEAN PATENT SPECIFICATION

(11) **EP 1 335 727 B1**
(45) Date of publication and mention of the grant of the patent: **06.10.2004**
(21) Application number: 01977093.2
(22) Date of filing: 12.10.2001
(51) Int. Cl.: A61K 31/445

(54) **METHOD FOR INHIBITING CATARACTS**
VERFAHREN ZUR INHIBIERUNG VON KATARAKTEN
PROCEDE POUR INHIBER LA CATARACTE

(30) Priority: 25.10.2000 US 243101 P; 29.11.2000 US 253800 P
(43) Date of publication of application: 20.08.2003
(73) Proprietor: ELI LILLY AND COMPANY, Indianapolis, Indiana 46285 (US)
(72) Inventor: ANDERSON, Pamela, Wang, Indianapolis, IN 46234 (US); SASHEGYI, Andreas, Indianapolis, IN 46202 (US)
(74) Representative: Burnside, Ivan John
(86) International application number: PCT/US2001/027766
(87) International publication number: WO 2002/034266

(56) References cited:
- EP-A- 0 662 325
- US-A- 5 641 790
- US-A- 5 747 510

## Description

The term "cataract" is a general term for any pathological condition in which the normal transparency of the ocular lens is substantially diminished. More than one million cataract extractions are performed annually in the United States, and it is estimated that 5 to 10 million individuals become visually disabled each year due to cataracts.

Although often regarded as an inevitable accompaniment of advancing age, cataracts may develop at any time in life, even before birth. Risk factors for cataract formation include metabolic disorders (e.g., diabetes), exposure to toxic agents in the environment (e.g., ultraviolet radiation, ionizing radiation), drug side effects, and inherited traits. Clinical experience suggests that the natural course of different types of cataracts are distinct. However, objective, quantitative data is generally lacking.

Development of anti-cataract agents has been hampered, in part, by the lack of a good animal model of human cataract. Consequently, putative anti-cataract agents may be evaluated for efficacy in a variety of different models which, to the extent that they are understood at all, are thought to occur by different mechanisms. For example, radiation-induced cataract is generally believed to result from oxidative damage to the lens. Diabetic cataract is thought to be due to the accumulation of polyols (such as sorbitol) in the lens, resulting from increased activity of the enzyme aldose reductase. According to The Royal College of Surgeons (RCS), hereditary cataract is thought to be due to the action of products released by the retina.

In contrast to the understanding of cataract pathogenesis, the cellular structure of the lens is fairly well characterized. The lens exhibits a high degree of regularity, consisting of fiber cells with hexagonal cross sections packed together to create a very regular parallel array of fiber cells which stretch from anterior to posterior pole. The lens fiber cells lose all intracellular organelles that could contribute to light scattering during the process of differentiation and the cytoplasmic protein concentration increases markedly.

Approximately 35% to 60% of the total mass of the lens consists of structural proteins, the remainder being water. More than 90% of the total lens protein consists of alpha, beta, and gamma crystallins, a group of structural proteins found at extremely high concentrations (in excess of 300 mg/ml) in the lens cell cytoplasm. The cytoplasmic concentration of the crystallins throughout the lens occurs along a continuous radial concentration gradient, in which the concentration is greatest in cells at the nucleus and decreases in peripheral cells of the lens cortex. The crystallin distribution determines the mean index of refraction and index gradient, which are in turn responsible for the special optical properties of the animal lens.

An important optical property is lens transparency. In the normal lens, incident light is scattered in all directions by the macromolecular constituents of the lens. If the individual wavelets of the scattered light interfere destructively with one another, the lens is transparent. Destructive interference takes place in the normal lens because of the existence of short range order in the relative positions of the crystallins. If the uniformity of the protein concentration is sufficiently perturbed, a substantial fraction of the incident light is scattered in directions away from the forward direction. The scattering results in a distortion of the wave front of the transmitted light, and in opacity of the lens tissue. The opacity is responsible for visual impairment in cataract diseases.

Cataracts are the leading cause of blindness in humans worldwide, and surgery remains the primary form of treatment. Cataracts in animals also pose a significant veterinary problem. To date, a compound for *in vivo* administration to humans or other animals has not been demonstrated to prevent cataracts of diverse origin. Further, *in vivo* reversal of the initiation of cataract formation has not been successfully demonstrated.

Therefore, there is a significant need for an effective nonsurgical method for treating or preventing cataractogenesis in humans and other animals. This method should utilize compounds which are relatively safe and which may be conveniently administered. The present invention fulfills these needs and provides other related advantages.

This invention relates to the use of a compound of the formula 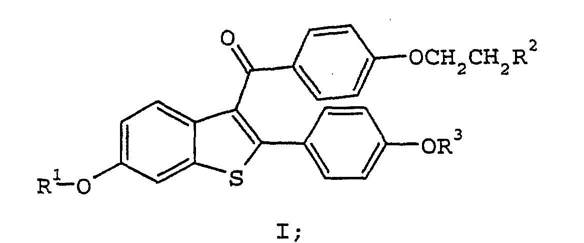 or a pharmaceutical salt or solvate thereof wherein:
R¹ and R³ are independently hydrogen, methyl, benzoyl, substituted benzoyl, or C(O)-(C₁-C₆ alkyl);
R² is selected from the group pyrolidin-1-yl, piperidin-1-yl, and hexamethyleneimin-1-yl; where the R² group is optionally the N-oxide for the preparation of a medicament for inhibiting cataracts.

As used herein, the term "inhibit" is defined to include its generally accepted meaning which includes preventing, prohibiting, restraining, and slowing, stopping or reversing progression, or severity, and holding in check and/or treating existing characteristics. The present method includes both medical therapeutic and/or prophylactic treatment, as appropriate.

The term "estrogen deficient" refers to a condition, either naturally occurring or clinically induced, where a woman can not produce sufficient estrogenic hormones to maintain estrogen dependent functions, *e.g.*, menses, homeostasis of bone mass, neuronal function, cardiovascular condition, *etc.* Such estrogen deficient situations arise from, but are not limited to, menopause and surgical or chemical ovarectomy, including its functional equivalent, *e.g.,* medication with GnRH agonists or antagonists, ICI 182780, and the like.

The term "patient" refers to a warm-blooded animal or mammal. It is understood that guinea pigs, dogs, cats, rats, mice, hamsters, rabbits and primates, including humans, are examples of patients within the scope of the meaning of the term.

General terms used in the description of compounds herein described bear their usual meanings. For example, "C₁-C₆ alkyl" refers to straight or branched aliphatic chains of 1 to 6 carbon atoms including methyl, ethyl, propyl, iso-propyl, n-butyl, pentyl, hexyl and the like.

The term "substituted phenyl" refers to a phenyl group alone or having one or more substituents selected from the group consisting of C₁-C₄ alkyl, C₁-C₄ alkoxy, hydroxy, nitro, chloro, fluoro, or tri(chloro or fluoro)methyl. "OC₁-C₄ alkyl" refers a C₁-C₄ alkyl group attached through an oxygen bridge such as , methoxy, ethoxy, n-propoxy, iso-propoxy, and the like.

The term "substituted benzoyl" refers to benzoyl group having one to five substituents selected independently from the group: C₁-C₄ alkyl, C₁-C₄ alkoxy, hydroxy, nitro, chloro, fluoro, or tri(chloro or fluoro)methyl.

The term "pharmaceutical salt" refers to either acid or base addition salts which are known to be non-toxic and are commonly used in the pharmaceutical literature. Commonly used acid addition salts are inorganic salts formed by the addition of sulfuric acid, nitric acid, hydrochloric acid, hydrobromic acid phosphoric acid, phosphorous acid and the like; or organic salts formed by the addition of acetic acid, formic acid, benzoic acid, citric acid, methanesulfonic acid and the like. Commonly used basic addition salts are the salts formed by alkali or alkaline earth hydroxides, ammonium hydroxide, alkyl or aromatic amines and the like. A preferred salt of this invention is the hydrochloride salt.

The term "solvate" refers to a molecular complex of a compound of formula I with one or more solvent molecules. Such solvent molecules would be those commonly used in the pharmaceutical literature, which are known to be innocuous to the recipient, e.g., water, ethanol, and the like.

The compounds of this invention are derivatives of centrally located carbon, i.e., the "-CO-" moiety in formula I, thus derivatives are methanones, e.g., a compound of A-CO-B, would be named [A][B]methanone. Further the compounds of formula I are derivatives of benzo[b]thiophene which is named and numbered according to the Ring Index, The American Chemical Society, as follows:

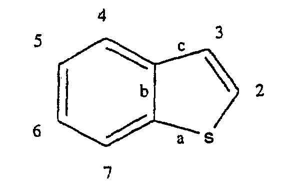

Thus, raloxifene hydrochloride, which is a preferred embodiment of this invention, is a compound of formula I, where R¹ and R³ are both hydrogen and R² is a piperidinyl ring, the hydrochloride salt thereof. Raloxifene hydrochloride is named [2-(4-hydroxyphenyl)-6-hydroxybenzo[b]thie-3-yl][4-[2-(1-piperidenyl)ethoxy]-phenyl]methanone hydrochloride. It is sold commercially as EVISTA® and is indicated in the United States for the prevention and treatment of osteoporosis.

The compounds of Formula I and salts and solvates thereof may be prepared according to known procedures, such as those detailed in U.S. Pat. Nos. 4,133,814, 4,418,068, 5,631,369, 5,731,327, 5,731,342, 5,750,688 and 5,977,383, each of which is incorporated by reference herein as if fully set forth. Preferred crystalline forms, particle sizes and pharmaceutical formulations are disclosed in U.S. Pat. Nos. 5,641,790, 5,731,327, 5,747,510, and 5,811,120.

The compounds of formula I are members of a group of compounds previously known as antiestrogens, but which have selective estrogenic agonist and antagonist pharmacologic activities. For example, formula I compounds act as estrogen agonists in treating pathologic sequelae caused by the cessation of menses in females (see: Draper *et al*., "Effects of Raloxifene (LY139481 HCl) on Biochemical Markers of Bone and Lipid Metabolism in Healthy Postmenopausal Women", Hong Kong, Fourth Int'l. Symp. on Osteoporosis, March 29, 1993.; US Pat. Nos. 5,393,763, 5,464,845, and 5,391,557).

Pharmaceutical formulations can be prepared by procedures known in the art, such as, for example, in European Published Application 670162A1, published September 6, 1995, and in WO 97/35571 published October 2, 1997. For example, a compound of formula I can be formulated with common excipients, diluents, or carriers, and formed into tablets, capsules, and the like.

Examples of excipients, diluents, and carriers that are suitable for formulation include the following: fillers and extenders such as starch, sugars, mannitol, and silicic derivatives; binding agents such as carboxymethyl cellulose and other cellulose derivatives, alginates, gelatin, and polyvinyl pyrrolidone; moisturizing agents such as glycerol; disintegrating agents such as agar, calcium carbonate, and sodium bicarbonate; agents for retarding dissolution such as paraffin; resorption accelerators such as quaternary ammonium compounds; surface active agents such as cetyl alcohol, glycerol monostearate; adsorptive carriers such as kaolin and bentonire; and lubricants such as talc, calcium and magnesium stearate and solid polyethyl glycols. Final pharmaceutical forms may be: pills, tablets, powders, lozenges, syrups, aerosols, saches, cachets, elixirs, suspensions, emulsions, ointments, suppositories, sterile injectable solutions, or sterile packaged powders, depending on the type of excipient used.

Additionally, raloxifene and its pharmaceutically acceptable salts are suited to formulation as sustained release dosage forms. The formulations can also be so constituted that they release the active ingredient only or preferably in a particular part of the intestinal tract, possibly over a period of time. Such formulations would involve coatings, envelopes, or protective matrices which may be made from polymeric substances or waxes.

As used herein, the term "effective amount" means an amount of compound of the present invention which is capable of inhibiting cataracts in a patient, preferably a human, and most preferably a post-menopausal woman.

The particular dosage of raloxifene or a pharmaceutically acceptable salt thereof required to constitute an effective amount according to this invention will depend upon the particular circumstances of the conditions to be treated. Considerations such as dosage, route of administration, and frequency of dosing are best decided by the attending physician. Generally, accepted and effective dose ranges for oral or parenteral administration will be from 10 mg to 800 mg, and more typically between 20 mg and 100 mg. Furthermore, an effective minimum dose for oral or parenteral administration of raloxifene or a pharmaceutically acceptable salt thereof is about 1, 5, 10, 15, or 20 mg. Typically, an effective maximum dose is about 800, 120, 60, 50, or 40 mg. A typical dosage range may be any combination of the above specific minimum and maximum doses. A particularly effective amount is 60 mg of raloxifene hydrochloride (56 mg of free base) per day via an oral route of administration. Another particularly effective amount is 120 mg of raloxifene hydrochloride per day via an oral route of administration. Such dosages will be administered to a patient in need of treatment from one to three times each day or as often as needed to effectively inhibit cataracts. Raloxifene hydrochloride may be administered for extended periods of time including six months to two years, specifically including about one year. Raloxifene hydrochloride may be used for repeated courses or continuously for an indefinite time.

The formulations which follow are given for purposes of illustration and are not intended to be limiting in any way. The total active ingredient in such formulations comprises from 0.1% to 99.9% by weight of the formulation. The term, "active ingredient" means a compound of formula I, or a pharmaceutical salt or solvate thereof, (preferably raloxifene hydrochloride). An even more preferred formulation of a compound of formula I would be raloxifene hydrochloride in the particular crystalline form, particle size, and composition illustrated in U.S. Pat. No. 5,731,327 and PCT application WO 97/35571 (2 October 1997).

### Formulation 1

| Gelatin Capsules | |
|---|---|
| Ingredient | Quantity (mg/capsule) |
| Active Ingredient | 50-600 |
| Starch NF | 0-500 |
| Starch flowable powder | 0-500 |
| Silicone fluid 350 centistrokes | 0-15 |

The ingredients are blended, passed through a No. 45 mesh U.S. sieve, and filled into hard gelatin capsules.

### Formulation 2

| Tablets | |
|---|---|
| Ingredient | Quantity (mg/tablet) |
| Active Ingredient | 50-600 |
| Starch | 10-50 |
| Cellulose, microcrystalline | 10-20 |
| Polyvinylpyrrolidone (as 10% solution in water) | 5 |
| Sodium carboxymethyl cellulose | 5 |
| Magnesium stearate | 1 |
| Talc | 1-5 |

The active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules thus produced are dried at 50-60° C and passed through a No. 18 mesh U.S. sieve. The sodium carboxymethyl cellulose, magnesium stearate, and talc, previously passed through a No. 60 mesh U.S. sieve, are added to the above granules and thoroughly mixed. The resultant material is compressed in a tablet forming machine to yield the tablets.

### Formulation 3

| Aerosol | |
|---|---|
| Ingredient | Weight % |
| Active Ingredient | 0.50 |
| Ethanol | 29.50 |
| Propellant 22 | 70.00 |
| (Chlorodifluoromethane) | |

The active ingredient is mixed with ethanol and the mixture added to a portion of the propellant 22, cooled to - 30°C and transferred to a filling device. The required amount is then fed to a stainless steel container and diluted with the remainder of the propellant. The valve units are then fitted to the container.

### Formulation 4

| Suspension | |
|---|---|
| Ingredient | Weight/Volume |
| Active Ingredient | 100 mg |
| Sodium carboxymethyl cellulose | 50 mg |
| Syrup | 1.25 mL |
| Benzoic acid solution (0.1M) | 0.10 mL |
| Flavor | q.v. |
| Color | q.v. |
| Purified water to total | 5 mL |

Suspensions each containing 100 mg of a compound of formula I per 5 mL dose are prepared as follows: the active ingredient is passed through a No. 45 mesh U.S. sieve and mixed with the sodium carboxymethyl cellulose and syrup to form a smooth paste. The benzoic acid solution, flavor, and color diluted in water are added and mixture stirred thoroughly. Additional water is added to bring the entire mixture to the required volume.

### Example

A phase 3, multicenter, double-blind, placebo-controlled, randomized clinical trial was conducted. The trial included 7705 women (mean age 67 years old), who were an average of 19 years postmenopausal. These patients were randomly assigned to the following treatment protocol: raloxifene hydrochloride at 60 mg per day via oral administration, raloxifene at 120 mg per day, or placebo. The study was conducted for a period of 48 months. As a portion of the patient's overall evaluation, various clinical tests and parameters were measured at periodic intervals. An analysis of adverse events relating to cataracts reported in this trial revealed a trend toward decreasing risk of cataracts with raloxifene, as compared to placebo. Furthermore, this trend appears to be dose dependent, with patients on the 120 mg per day dose experiencing fewer events than patients on the 60 mg per day dose, and patients on both doses experiencing fewer events than those on placebo.

The following tables provide summaries of select cataract treatment-emergent signs and symptoms ("TESS") events for various patient populations at the thirty-six and the forty-eight month mark of the clinical trial.

**TABLE 1:**

| **All Randomized Patients - 36-Month Data** | | | | | |
|---|---|---|---|---|---|
| **Event Classification** | **Placebo** | **R1x60** | **R1x120** | **Comparison** | **p-value** |
| Any Cataract-related event | (N=2576) 124 (4.8%) | (N=2557) 105 (4.1%) | (N=2572) 101 (3.9%) | Overall | 0.252 |
| | | | | Pla v. Rlx | 0.103 |
| | | | | Pla v. 060 | 0.220 |
| | | | | Pla v. 120 | 0.120 |
| | | | | 060 v. 120 | 0.743 |
| "Cataract Specified" | (N=2576) 110 (4.3%) | (N=2557) 92 (3.6%) | (N=2572) 82 (3.2%) | Overall | 0.115 |
| | | | | Pla v. Rlx | 0.054 |
| | | | | Pla v. 060 | 0.216 |
| | | | | Pla v. 120 | 0.041 |
| | | | | 060 v. 120 | 0.418 |
| Surgery for cataract | (N=2576) 56 (2.2%) | (N=2557) 60 (2.3%) | (N=2572) 60 (2.3%) | Overall | 0.899 |
| | | | | Pla v. Rlx | 0.646 |
| | | | | Pla v. 060 | 0.677 |
| | | | | Pla v. 120 | 0.701 |
| | | | | 060 v. 120 | 0.974 |

**TABLE 2:**

| **Patients Reporting a Cataract-Related Current Condition at Baseline - 36-Month Data** | | | | | |
|---|---|---|---|---|---|
| **Event Classification** | **Placebo** | **R1x60** | **R1x120** | **Comparison** | **p-value** |
| Any Cataract-related event | (N=170) 28 (16.5%) | (N=163) 25 (15.3%) | (N=154) 18(11.7%) | Overall | 0.450 |
| | | | | Pla v. Rlx | 0.386 |
| | | | | Pla v. 060 | 0.778 |
| | | | | Pla v. 120 | 0.218 |
| | | | | 060 v. 120 | 0.343 |

**TABLE 3:**

| **Patients Not Reporting a Cataract-Related Current Condition at Baseline - 36-Month Data** | | | | | |
|---|---|---|---|---|---|
| **Event Classification** | **Placebo** | **R1x60** | **R1x120** | **Comparison** | **p-value** |
| Any Cataract-related event | (N=2406) 96 (4.0%) | (N=2394) 80 (3.3%) | (N=2418) 83 (3.4%) | Overall | 0.425 |
| | | | | Pla v. Rlx | 0.194 |
| | | | | Pla v. 060 | 0.232 |
| | | | | Pla v. 120 | 0.306 |
| | | | | 060 v. 120 | 0.862 |

**TABLE 4:**

| **All Randomized Patients - 48-Month Data** | | | | | |
|---|---|---|---|---|---|
| **Event Classification** | **Placebo** | **R1x60** | **R1x120** | **Comparison** | **p-value** |
| Any Cataract-related event | (N=2576) 160 (6.2%) | (N=2557) 151 (5.9%) | (N=2572) 140(5.4%) | Overall | 0.498 |
| | | | | Pla v. Rlx | 0.343 |
| | | | | Pla v. 060 | 0.646 |
| | | | | Pla v. 120 | 0.240 |
| | | | | 060 v. 120 | 0.474 |
| "Cataract Specified" | (N=2576) 141 (5.5%) | (N=2557) 135 (5.3%) | (N=2572) 122(4.7%) | Overall | 0.472 |
| | | | | Pla v. Rlx | 0.386 |
| | | | | Pla v. 060 | 0.758 |
| | | | | Pla v. 120 | 0.234 |
| | | | | 060 v. 120 | 0.379 |
| Surgery for cataract | (N=2576) 86 (3.3%) | (N=2557) 85 (3.3%) | (N=2572) 78 (3.0%) | Overall | 0.783 |
| | | | | Pla v. Rlx | 0.707 |
| | | | | Pla v. 060 | 0.977 |
| | | | | Pla v. 120 | 0.532 |
| | | | | 060 v. 120 | 0.552 |

**TABLE 5:**

| **Patients Reporting a Cataract-Related Current Condition at Baseline - 48-Month Data** | | | | | |
|---|---|---|---|---|---|
| **Event Classification** | **Placebo** | **R1x60** | **R1x120** | **Comparison** | **p-value** |
| Any Cataract-related event | (N=176) 33 (18.8%) | (N=172) 31 (18.0%) | (N=164) 25(15.2%) | Overall | 0.670 |
| | | | | Pla v. Rlx | 0.555 |
| | | | | Pla v. 060 | 0.861 |
| | | | | Pla v. 120 | 0.390 |
| | | | | 060 v. 120 | 0.494 |

**TABLE 6:**

| **Patients Not Reporting a Cataract-Related Current Condition at Baseline - 48-Month Data** | | | | | |
|---|---|---|---|---|---|
| **Event Classification** | **Placebo** | **R1x60** | **R1x120** | **Comparison** | **p-value** |
| Any Cataract-related event | (N=2400) 127 (5.3%) | (N=2385) 120 (5.0%) | (N=2408) 115 (4.8%) | Overall | 0.716 |
| | | | | Pla v. Rlx | 0.477 |
| | | | | Pla v. 060 | 0.684 |
| | | | | Pla v. 120 | 0.413 |
| | | | | 060 v. 120 | 0.682 |

While the effect of estrogen on cataracts has not been completely described to date, investigators have noted in limited observational trials that use of oral estrogen in postmenopausal women is associated with a seeming reduction in the incidence of cataracts (Klein BE, Klein R, Ritter LL. Is there evidence of an estrogen effect on age-related lens opacities? The Beaver Dam Eye Study (see comments]. Arch Ophthal 112:85-91, 1994). Unfortunately, to date, the largest prospective clinical trial of oral estrogen (the HERS trial), was too small to see an effect of estrogen on cataracts (Hulley S, Grady D, Bush T, Furberg C, Herrington D, Riggs B, Vittinghoff. Randomized trial of estrogen plus progestin for secondary prevention of coronary heart disease in postmenopausal women. JAMA 280: 605-613, 1998). The mechanism for this seeming reduction in cataracts may be due to oral estrogen's ability to ameliorate the damaging effects of TGF-beta on the lens (Hales AM, Chamberlain CG, Murphy CR, McAvoy JW, Estrogen protects lenses against cataract induced by transforming growth factor-beta. J Exp Med 185:273-80, 1997.

With the exception of tamoxifen, the effect of SERMs on cataracts has not been reported. In the largest prospective clinical trial performed with tamoxifen to date (the NSABP's P-1 trial), tamoxifen was associated with a relative risk of cataracts of 1.14 with a borderline significant 95% CI of 1.01-1.29 (Fisher B, Costantino JP, Wickerham DL, Redmond CK, Kavanah M, Cronin WM, Vogel V, Robidoux A, Dimitrov N, Atkins J, Daly M, Wieand S, Tan-Chiu E, Ford L, Wolmark N. Tamoxifen for prevention of breast cancer: Report of the National Surgical Adjuvant Breast and Bowel Project P-1 Study. J Nat1 Cancer Inst 90: 1371-1388, 1998). Presumably, tamoxifen is acting as an anti-estrogen in the lens. Studies in animals have suggested that one possible mechanism by which tamoxifen increases the risk of cataracts may be by blocking chloride channels. Chloride channels in the lens are essential for maintaining normal lens hydration and transmittance. In organ culture, tamoxifen blocked chloride channels and led to lens opacity associated with cataracts at clinically relevant concentrations (Zhang JJ, Jacob TJ, Valverde MA, Hardy SP, Mintenig GM, Sepulveda FV, Gill DR, Hyde SC, Trezise AE, Higgins CF. Tamoxifen blocks chloride channels. A possible mechanism for cataract formation. J Clin Invest 94:1690-7, 1994).

It is reasonable to expect that tamoxifen and raloxifene, although they are both SERMs, have different effects on the lens, since they are of different chemical classes and have been shown to differ in their effects on at least one other organ system, namely, the uterus. Tamoxifen has been shown to cause endometrial stimulation and rarely, endometrial cancer Fisher B *et al*., *supra*. Raloxifene, on the other hand, is not associated with endometrial stimulation and has not been associated with endometrial cancer (Davies GC, Huster WJ, Shen W, Mitlak B, Plouffe L, Shah A, Cohen FJ. Endometrial response to raloxifene compared with placebo, cyclical hormone replacement therapy and unopposed estrogen in postmenopausal women. Menopause 6:188-195, 1999 and Goldstein SR, Scheele WH, Rajagopalan SK, Wilkie J, Walsh BW, Parsons AK. A 12-month comparative study of raloxifene, estrogen and placebo on the postmenopausal endometrium. Obstet Gynecol 95: 95-103, 2000 and Cohen FJ, Watts S, Shah A, Akers R, Plouffe L. Uterine effects of 3-year raloxifene therapy in postmenopausal women younger than age 60. Obstet Gynecol 95:104-110, 2000).

## Claims

1. Use of a compound of the formula 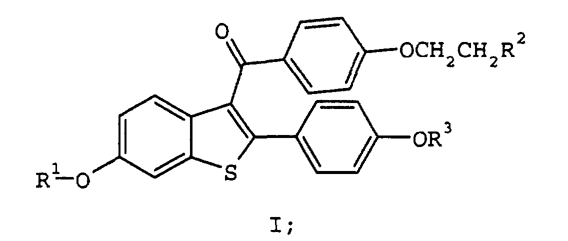 or a pharmaceutical salt or solvate thereof wherein:
R¹ and R³ are independently hydrogen, methyl, benzoyl, substituted benzoyl, or C(O)-(C₁-C₆ alkyl);
R² is selected from the group pyrolidin-1-yl, piperidin-1-yl, and hexamethyleneimin-1-yl; where the R² group is optionally the N-oxide, in the preparation of a medicament for inhibiting cataracts.

2. Use according to Claim 1 wherein said medicament is adapted for human use.

3. Use according to Claim 2 wherein said human is a female.

4. Use according to Claim 3 wherein said female is estrogen deficient.

5. Use according to Claims 1 to 4 wherein said compound of formula I is a pharmaceutical acid addition salt, R¹ and R³ are hydrogen, and R² is piperidin-1-yl.

6. Use according to Claims 1 to 5 wherein said compound of formula I is the hydrochloride salt.

7. Use according to Claims 1 to 4 wherein said compound of formula I is a pharmaceutical acid addition salt, R¹ and R³ are hydrogen, and R² is pyrolidin-1-yl.

8. Use according to Claim 7 wherein said compound of formula I is the hydrochloride salt.

## Patentansprüche

1. Verwendung einer Verbindung der folgenden Formel 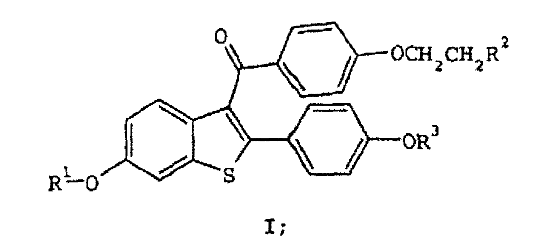 oder eines pharmazeutischen Salzes oder Solvats davon, worin:
R¹ und R³ unabhängig voneinander stehen für Wasserstoff, Methyl, Benzoyl, substituiertes Benzoyl oder C(O)-(C₁-C₆-Alkyl);
R² ausgewählt ist aus der Gruppe von Pyrrolidin-1-yl, Piperidin-1-yl und Hexamethylenimin-1-yl; wobei die R²-Gruppe gegebenenfalls das N-oxid ist, bei der Herstellung eines Medikaments zur Inhibition oder Hemmung von Katarakten.

2. Verwendung gemäß Anspruch 1, wobei das Medikament eingerichtet ist zur Verwendung am Menschen.

3. Verwendung gemäß Anspruch 2, wobei der Mensch eine Frau ist.

4. Verwendung gemäß Anspruch 3, wobei die Frau einen Mangel an Östrogen aufweist.

5. Verwendung gemäß Anspruch 1 bis 4, wobei die Verbindung der Formel I ein pharmazeutisches Säureadditionssalz ist, R¹ und R³ für Wasserstoff stehen, und R² für Piperidin-1-yl steht.

6. Verwendung gemäß der Ansprüche 1 bis 5, wobei die Verbindung der Formel I das Hydrochloridsalz ist.

7. Verwendung gemäß der Ansprüche 1 bis 4, wobei die Verbindung der Formel I ein pharmazeutisches Säureadditionssalz ist, R¹ und R³ für Wasserstoff stehen, und R² für Pyrrolidin-1-yl steht.

8. Verwendung gemäß Anspruch 7, wobei die Verbindung der Formel I das Hydrochloridsalz ist.

## Revendications

1. Utilisation d'un composé de formule : 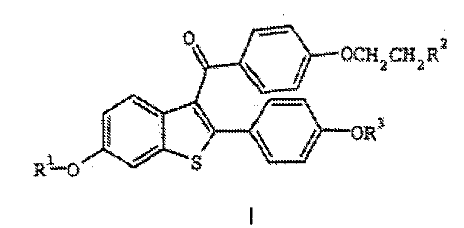 ou d'un sel ou d'un solvate pharmaceutique de celui-ci, dans laquelle :
R¹ et R³ représentent indépendamment un atome d'hydrogène, un groupe méthyle, un groupe benzoyle, un groupe benzoyle substitué, ou un groupe C(O) - (alkyle en (C₁-C₆)) ;
R² est choisi parmi les groupes pyrrolidin-1-yle, pipéridin-1-yle, et hexaméthylèneimin-1-yle ; où le groupe R² représente éventuellement l'oxyde d'azote, dans la préparation d'un médicament pour inhiber les cataractes.

2. Utilisation selon la revendication 1, dans laquelle ledit médicament est adapté à une utilisation chez les humains.

3. Utilisation selon la revendication 2, dans laquelle ledit humain est une femme.

4. Utilisation selon la revendication 3, dans laquelle ladite femme est carencée en oestrogènes.

5. Utilisation selon les revendications 1 à 4, dans laquelle ledit composé de formule I est un sel pharmaceutique d'addition d'un acide, R¹ et R³ représentent un atome d'hydrogène, et R² représente un groupe pipéridin-1-yle.

6. Utilisation selon les revendications 1 à 5, dans laquelle ledit composé de formule I est le sel hydrochlorure.

7. Utilisation selon les revendications 1 à 4, dans laquelle ledit composé de formule I est un sel pharmaceutique d'addition d'un acide, R¹ et R³ représentent un atome d'hydrogène, et R² représente un groupe pyrrolidin-1-yle.

8. Utilisation selon la revendication 7, dans laquelle ledit composé de formule I est le sel hydrochlorure.
